# EUROPÄISCHE PATENTANMELDUNG

(11) **EP 2 196 093 A1**
(43) Veröffentlichungstag der Anmeldung: **16.06.2010**
(21) Anmeldenummer: 08021731.8
(22) Anmeldetag: 15.12.2008
(51) Int. Cl.: A01N 63/00, C12N 9/02, C12P 1/04, C12P 7/04, C12R 1/01

(54) **Verbesserung des Erdbeeraromas**

(71) Anmelder: Technische Universität Graz, 8010 Graz (AT); Forschungsholding TU Graz GmbH, 8010 Graz (AT)
(72) Erfinder: Berg, Gabriele, 8010 Graz (AT); Müller, Henry, 8010 Graz (AT); Leitner, Erich, 8010 Graz (AT); Siegmund, Barbara, 8010 Graz (AT)
(74) Vertreter: Sonn & Partner Patentanwälte

(57) **Zusammenfassung**

Beschrieben wird ein Präparat enthaltend Isolate, Derivate oder Mutanten von RAB-1 und/oder SAB-1, hinterlegt unter den Hinterlegungsnummern DSM21961 und DSM21962 bei der Hinterlegungsstelle DSMZ, wobei die Isolate, Derivate oder Mutanten in Form von lebenden Zellen, toten Zellen und/oder Fragmenten davon und/oder aus Kulturüberständen derselben besteht. Dieses Präparat kann zur Geschmacksverbesserung der Erdbeere verwendet werden.

SEQUENCE LISTING

<110>  Technische Universität Graz

<120>  Verbesserung des Erdbeeraromas

<130>  R 52754

<160>  5

<170>  PatentIn version 3.4

<210>  1
<211>  1381
<212>  DNA
<213>  Methylobacterium extorquens

<400>  1
aggggggctt accatgcaag tcgacgggca ccttcgggtg tcagtggcag acgggtgagt      60

aacacgtggg aacgtaccct tcggttcgga ataactcagg gaaacttgag ctaataccgg     120

atacgccctt ttggggaaag gtttactgcc gaaggatcgg cccgcgtctg attagcttgt     180

tggtggggta acggcctacc aaggcgacga tcagtagctg gtctgagagg atgatcagcc     240

acactgggac tgagacacgg cccagactcc tacgggaggc agcagtgggg aatattggac     300

aatgggcgca agcctgatcc agccatgccg cgtgagtgat gaaggcctta gggttgtaaa     360

gctcttttgt ccgggacgat aatgacggta ccggaagaat aagccccggc taacttcgtg     420

ccagcagccg cggtaatacg aagggggcta gcgttgctcg gaatcactgg gcgtaaaggg     480

cgcgtaggcg gccgattaag tcgggggtga aagcctgtgg ctcaaccaca gaattgcctt     540

cgatactggt tggcttgaga ccggaagagg acagcggaac tgcgagtgta gaggtgaaat     600

tcgtagatat tcgcaagaac accagtggcg aaggcggctg tctggtccgg ttctgacgct     660

gaggcgcgaa agcgtgggga gcaaacagga ttagataccc tggtagtcca cgccgtaaac     720

gatgaatgcc agccgttggc ctgcttgcag gtcagtggcg ccgctaacgc attaagcatt     780

ccgcctgggg agtacggtcg caagattaaa actcaaagga attgacgggg gcccgcacaa     840

gcggtggagc atgtggttta attcgaagca acgcgcagaa ccttaccatc ccttgacatg     900

gcatgttacc tcgagagatc ggggatcctc ttcggaggcg tgcacacagg tgctgcatgg     960

ctgtcgtcag ctcgtgtcgt gagatgttgg gttaagtccc gcaacgagcg caacccacgt    1020

ccttagttgc catcatttag ttgggcactc tagggagact gccggtgata agccgcgagg    1080

aaggtgtgga tgacgtcaag tcctcatggc ccttacggga tgggctacac acgtgctaca    1140

atggcggtga cagtgggacg cgaagccgcg aggtggagca aatccccaaa aaccgtctca    1200

gttcggattg cactctgcaa ctcgggtgca tgaaggcgga atcgctagta atcgtggatc    1260

agcacgccac ggtgaatacg ttcccgggcc ttgtacacac cgcccgtccc cccatgggag    1320

ttggtcttac ccgacggcgc tgcgccaacc gcaaggaggg caggcgacca cgtagttacg    1380

a                                                                    1381

<210>  2
<211>  1420
<212>  DNA

<213>  Methylobacterium mesophilicum

<400>  2
ttggacctcc gtggtcgcct gcctccttgc ggttggcgca gcgccgtcgg gtaagaccaa      60

ctcccatggg gggacgggcg gtgtgtacaa ggcccgggaa cgtattcacc gtggcatgct     120

gatccacgat tactagcgat tccgccttca tgcacccgag ttgcagagtg caatccgaac     180

tgagacggct tttggggatt tgctccaggt cgccccttcg cgtcccactg tcaccgccat     240

tgtagcacgt gtgtagccca tcccgtaagg gccatgagga cttgacgtca tccacacctt     300

cctcgcggct tatcaccggc agtctcccta gagtgcccaa ctgaatgatg gcaactaagg     360

acgtgggttg cgctcgttgc gggacttaac ccaacatctc acgacacgag ctgacgacag     420

ccatgcagca cctgtgtgcg cgccaccgaa gtggactggg aatctctccc cataacacgc     480

catgtcaaag gatggtaagg ttctgcgcgt tgcttcgaat taaaccacat gctccaccgc     540

ttgtgcgggc ccccgtcaat tcctttgagt tttaatcttg cgaccgtact ccccaggcgg     600

aatgctcaaa gcgttagctg cgctactgag gtgcaagcac cccaacagct ggcattcatc     660

gtttacggcg tggactacca gggtatctaa tcctgtttgc tccccacgct ttcgcgcctc     720

agcgtcagta atggtccagt tggccgcctt cgccaccggt gttcttgcga atatctacga     780

atttcacctc tacactcgca gttccaccaa cctctaccat actcaagcgt cccagtatcg     840

aaggccattc tgtggttgag ccacaggctt tcacccccga cttaaaacgc cgcctacgcg     900

ccctttacgc ccagtgattc cgagcaacgc tagccccctt cgtattaccg cggctgctgg     960

cacgaagtta gccggggctt attcctccgg taccgtcatt atcgtcccgg ataaaagagc    1020

tttacaaccc taaggccttc atcactcacg cggcatggct ggatcaggct tgcgcccatt    1080

gtccaatatt ccccactgct gcctcccgta ggagtctggg ccgtgtctca gtcccagtgt    1140

ggctgatcat cctctcagac cagctactga tcgtcgcctt ggtgagccgt tacctcacca    1200

actagctaat cagacgcggg ccgatcctcc ggcagcaagc ctttccccaa aagggcgtat    1260

ccggtattag ctcaagtttc cctgagttat tccgaaccag agggcacgtt cccacgcgtt    1320

actcacccgt ccgccgctga caccgaagga tgccccgctc gacttgcatg tgttgacccc    1380

tgccccccag cgttcgctct gagcgaggat acaatcctca                          1420

<210>  3
<211>  18
<212>  DNA
<213>  Artificial

<220>
<223>  primer

<400>  3
gagtttgatc ctggctca                                                    18

<210>  4
<211>  20
<212>  DNA
<213>  Artificial

<220>

<223>  Primer

<400>  4
agaaaggagg tgatccagcc                                                    20


<210>  5
<211>  22
<212>  DNA
<213>  Artificial

<220>
<223>  Primer

<400>  5
ctacggcaag gcgacgctga cg                                                 22

## Beschreibung

Die Erfindung betrifft Verfahren und Mittel zur Geschmacksverbesserung der Erdbeere.

Das typische Erdbeeraroma setzt sich aus insgesamt ca. 350 Substanzen zusammen. Dieser Fakt allein zeigt die Komplexität des Geschmacks der Erdbeerfrüchte. Zwei Substanzen, die chemisch zur Gruppe der Furanone zählen (2,5-Dimethyl-4-methoxy-2H-furan-3-on (DMMF bzw. Mesifuran) und 2,5-Dimethyl-4-hydroxy-2H-furan-3-on (DMHF bzw. Furaneol)), zählen zu den Verbindungen, die für die Ausbildung des typischen Erdbeeraromas am stärksten beitragen. 1,2-Propandiol, welches als Substanz von den Erdbeerpflanzen gebildet wird, dient hierbei als Vorstufe zur Synthese der beiden Substanzen. Pflanzenassoziierte Bakterien der Gattung Methylobacterium sind in der Lage, einen wesentlichen Schritt in der Biosynthese der beiden Substanzen, die Oxidation von 1,2-Propandiol zu 2-Hydroxypropanal, durchzuführen. Zabetakis (1997) veröffentlichte eine Studie, in der er nachwies, dass in Erdbeerzellkulturen diese beiden wichtigen Komponenten des Erdbeeraromas, DMMF und DMHF, nur unter Zusatz von Methylobacterium gebildet werden. Interessanterweise besitzt DMHF zusätzlich antimikrobielle Eigenschaften, welche auf der einen Seite die gesundheitsfördernden Aspekte der Erdbeere unterstreichen und auf der anderen Seite zeigen, dass die Bildung von DMHF potenziell als Abwehrreaktion der Pflanze verstanden werden kann.

Methylobakterien wurden als pflanzenassoziierte Bakterien erst vor ca. 20 Jahren entdeckt. Hierbei handelt es sich um eine Gruppe von Bakterien, die fakultativ methylotroph (d.h. von Methanol und verwandten Substanzen) leben, was für pflanzenassoziierte Bakterien sehr ungewöhnlich ist. Hierzu zählt auch ihr Phänotyp, der in der Regel verschiedene, intensive Rot-Töne hat, was ihnen auch den Namen "Pink Pigmented Facultative Methylotrophs" (PPFMs) verliehen hat. An der Pflanze sind PPFMs vorwiegend an Blättern, hier insbesondere in den Spaltöffnungen, aber auch im Inneren zu finden. Offensichtlich gehen Methylobakterien eine enge Interaktion mit Pflanzen ein, was auch ihr häufiges Vorkommen an den phylogenetisch sehr alten Moospflanzen erklärt. Für Letztere ist sogar bekannt geworden, dass zumindest einige Moosarten ohne die Assoziation mit Methylobacterium nicht keimen können. Auch für höhere Pflanzen sind derartige Effekte beschrieben, z.B. ein positiver Einfluss auf die Keimung und das Pflanzenwachstum (Lidstrom & Chistoserdova 2002). Methylobakterien produzieren ungewöhnlich viele pflanzliche Hormone, was ebenfalls für eine direkte Einflussnahme der Bakterien auf die Pflanzenentwicklung spricht.

Die Aufgabe der vorliegenden Erfindung ist, Verfahren und Mittel zur Geschmacksverbesserung der Erdbeere zur Verfügung zu stellen.

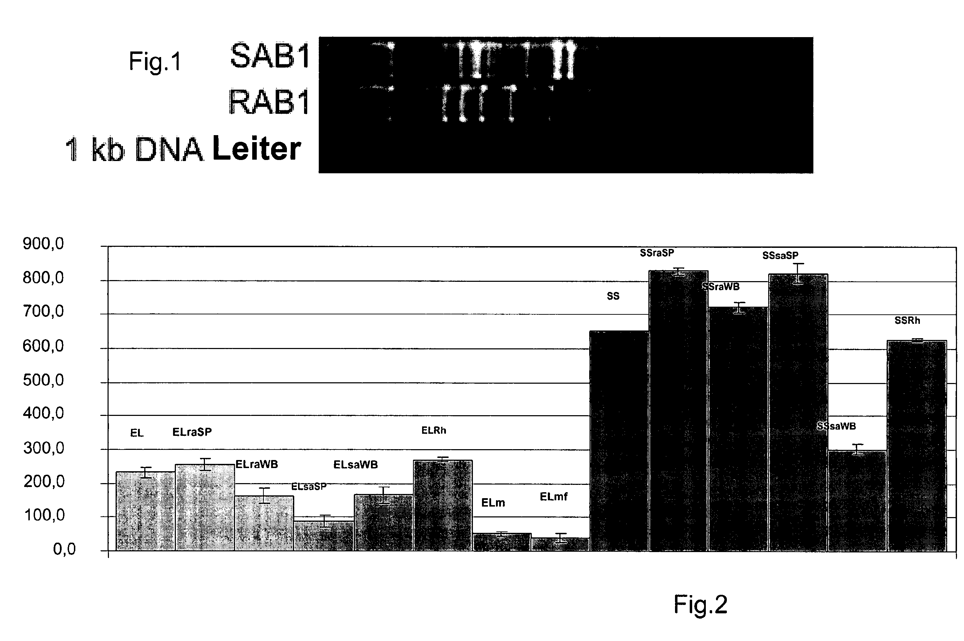

Demgemäß betrifft die vorliegende Erfindung das Isolat Methylobacterium RAB-1, hinterlegt am 29. Oktober 2008 unter der Hinterlegungsnummer DSM21961 bei der Hinterlegungsstelle DSMZ (Deutsche Sammlung von Mikroorganismen und Zellkulturen), und Derivate sowie Mutanten davon, wobei die Derivate und Mutanten definiert sind wie in Madigan und Martinko, "Mikrobiologie" (2006), Pearson Studium. Insbesondere weisen die erfindungsgemäßen Methylobakterien identische BOX-Muster (vgl. Fig. 1) auf. Weiters betrifft die vorliegende Erfindung das Isolat Methylobacterium SAB-1, hinterlegt am 29. Oktober 2008 unter der Hinterlegungsnummer DSM21962 bei der Hinterlegungsstelle DSMZ, und Derivate sowie Mutanten davon, wobei die Derivate und Mutanten wie oben definiert sind (auch im Hinblick auf das BOX-Muster).

Die erfindungsgemäßen Bakterienisolate RAB-1 und SAB-1 gehören zur weltweit verbreiteten Bakteriengattung Methylobacterium. Zu dieser Gattung gehören natürlich vorkommende Pflanzenmikroorganismen, die vorwiegend eine positive Interaktion mit der Pflanze eingehen.

Es hat sich herausgestellt, dass die erfindungsgemäßen Isolate sowie die von den Isolaten abstammenden Derivate (also die im Wesentlichen unveränderten direkten Abkömmlinge der Original-Isolate) und Mutanten geeignet sind, die sensorischen Eigenschaften von Erdbeeren (das "Erdbeeraroma") zu verbessern bzw. zu intensivieren. Dabei enthalten "Derivate" im Wesentlichen dieselbe Erbinformation wie die ursprünglichen Isolate und sind durch allgemein zugängliche, natürliche Vermehrungsverfahren vermehrt worden. Unter Erbinformation werden im Folgenden insbesondere Gene, Gensequenzen oder das Genom des Isolats, der Derivate oder einer Mutante verstanden.

Die Isolate haben u.a. die in der folgenden Tabelle 1 dargestellten Eigenschaften:

**Tabelle 1: Eigenschaften der selektierten Mikroorganismen**

| Isolat | Herkunft | Identität | Wachstum 37 °C | Milchsäurealdehyd (mg 10⁻⁶ Zellen) |
|---|---|---|---|---|
| RAB-1 | Phyllosphäre einer Rosaceae | Methylobacterium extorquens | - | 854,27 |
| SAB-1 | Phyllosphäre von Galanthus nivalis | Methylobacterium mesophilicum | - | 497,04 |

Wie erwähnt, sind den erfindungsgemäßen Isolaten auch davon abgeleitete (unveränderte) Derivate und Mutanten davon gleichgestellt, wenn diese Mutanten und Derivate dieselben erfindungswesentlichen Eigenschaften aufweisen.

Weiters betrifft die vorliegende Erfindung Präparate enthaltend die erfindungsgemäßen Isolate, Derivate oder Mutanten, wobei die Isolate, Derivate oder Mutanten in Form von lebenden Zellen, toten Zellen und/oder Fragmenten davon und/oder aus Kulturüberständen derselben bestehen. Dabei hat sich erfindungsgemäß gezeigt, dass nicht nur die Bakterien selbst eingesetzt werden können, sondern die positiven Eigenschaften, vor allem in Bezug auf die Verbesserung des Erdbeeraromas, sich auch schon mit der Anwendung der toten Zellen, von Zellfragmenten oder gar Kulturüberständen ergeben.

Diese Präparate können vorzugsweise in einer Weise bereitgestellt werden, dass sie die erfindungsgemäßen Isolate, Derivate oder Mutanten in einer aromaverbessernden wirksamen Menge enthalten.

Dabei kann das erfindungsgemäße Präparat entweder RAB-1 oder SAB-1 in Reinform oder aber in Form von Gemischen enthalten.

Die praktische Anwendung der vorliegenden Erfindung liegt bevorzugterweise in der Kultivierung von Erdbeeren. Die Isolate bzw. Isolatgemische können formuliert sein, mittels Seed-Priming appliziert oder als Suspension im Sprühverfahren angewandt werden. Das erfindungsgemäße Präparat kann auch über Nährstoffe, Nährsubstrate oder Nährflüssigkeiten appliziert werden.

Gemäß einem weiteren Aspekt betrifft die vorliegende Erfindung ein Verfahren zur Aromaverbesserung der Erdbeere, welches sich dadurch auszeichnet, dass ein erfindungsgemäßes Präparat auf die Pflanzen oder Pflanzenteile, auf das Pflanzgut, den Samen und/oder in bzw. auf den Boden appliziert wird.

Das erfindungsgemäße Verfahren kann auch für andere Früchte Verwendung finden, so dass die Aromaverbesserung von anderen Früchten protegiert wird.

Besonders vorteilhaft ist erfindungsgemäß die Applikation des Präparats über eine natürliche oder künstliche Matrix.

Die Erfindung wird anhand der nachfolgenden Beispiele und den Zeichnungsfiguren, auf die sie selbstverständlich nicht beschränkt ist, näher erläutert.

Es zeigen:
Fig. 1: das BOX-Muster der wirksamen Isolate; und
Fig. 2: die gemessenen Mesifuranwerte als Äquivalente zu einem Mesifuranstandard (y-Achse: TCP-Äquivalente), EL = Sorte Elsanta, SS = Sorte Senga Sengana, Elm = Elsanta vom Markt, Elmf = Elsanta vom Markt bei -70°C tiefgefroren, ra = RAB-1, sa = SAB-1, Rh = Rhizostar, SP = Sprühbehandlung, WB = Wurzelbad. Die Sorte Senga Sengana hat höhere Mesifuranwerte als die Sorte Elsanta, die Elsanta vom Markt haben die niedrigsten Mesifuranwerte.

### Beispiele

### Beispiel 1: Identifizierung der wirksamen Isolate

Die wirksamen Isolate wurden durch ein Screeningverfahren aus einem Pool aus verschiedenen Methylobacterium-Stämmen selektiert. Dabei wurden die verschiedenen Stämme anhand ihres Potentials zur Bildung von 2-Hydroxypropanal gereiht.

### Methode: Produktion von Milchsaurealdehyd

Die Produktion von Milchsäurealdehyd war eines der wichtigsten Kriterien für eine Anwendung zur Aromasteigerung, da Milchsäurealdehyd ein Indikator für die Bildung von 2-Hydroxypropanal, die Vorstufe von zwei Hauptaromasubstanzen (2 Furanone, DMHF und der Methylether davon, Mesifuran) ist. Durch die Produktion von Milchsäurealdehyd durch die Bakterienzelle, das dann an die Erdbeerpflanzenzelle abgegeben wird, steht der Pflanzenzelle zusätzlich Milchsäurealdehyd zur Verfügung, aus dem sie dann enzymatisch die beiden Furanone synthetisieren kann. Die Fähigkeit zur Produktion der einzelnen Stämme wurde mit der HPLC (RP-C18) nachgewiesen und die produzierte Menge quantifiziert. Dazu wurde eine Methode zum Nachweis von Milchsäurealdehyd als 2,4-Dinitrophenylhydrazon für die HPLC genutzt.

Die beiden wirksamsten Isolate dieser Reihung wurden durch Sequenzierung der 16S rDNA und durch eine anschließende BLAST-Suche mit den erhaltenen Sequenzen in der NCBI-Datenbank identifiziert.

### Methode: Identifizierung der Isolate

Bakterielle DNA der beiden Stämme wurde zunächst mittels AR-DRA (Amplified Ribosomal DNA Restriction Analysis) charakterisiert und anschließend das 16S rDNA-Fragment sequenziert. Die kleine Untereinheit des rRNA-Gens wurde mit einem TGradient Thermocycler amplifiziert (Biometra, Göttingen, Deutschland). Das 50 µl Reaktionsgemisch enthielt 10 µl PCR SuperMIX Taq & Go, 1 µl of Primer EubI-forward (5'- GAG TTT GAT CCT GGC TCA G-3'; SEQ.ID.NO 3) und 2 µl EubII-reverse (5'- AGA AAG GAG GTG ATC CAG CC -3'; SEQ.ID.NO 4) sowie 2 µl Template. Die PCR Produkte wurden mit dem QIAquick Gel Extractionskit (QIAGEN, Hilden, Germany) gereinigt und sequenziert.

Die folgenden Sequenzen wurden ermittelt:
RAB-1 (Methylobacteruim extorquens; SEQ.ID.NO 1): 
5' AGGGGGGCTTACCATGCAAGTCGACGGCACCTTCGGGTGTCAGTGGCAGACGGGTGAGTAACACGTGGGAACGTACCCTTCGGTTCGGAA-
TAACTCAGGGAAACTTGAGCTAATACCGGATACGCCCTTTTGGGGAAAGGTTTACTGCCGAAGGATCGGCCCGCGTCTGATAGCTTGTTGGTGGGG-
TAACGGCCTACCAAGGCGACGATCAGTAGCTGGTCTGAGAGGATGATCAGCCACACTGGGACTGAGACACGGCCCAGACTCCTACGGGAGGCAGCA-
GTGGGGAATATTGGACAATGGGCGCAAGCCTGATCCAGCCATGCCGCGTGAGTGATGAAGGCCTTAGGGTTGTAAAGCTCTTTTGTCCGGGACGA-
TAATGACGGTACCGGAAGAATAAGCCCCGGCTAACTTCGTGCCAGCAGCCGCGGTAATACGAAGGGGGCTAGCGTTGCTCGGAATCACTGGGCG-
TAAAGGGCGCGTAGGCGGCCGATTAAGTCGGGGGTGAAAGCCTGTGGCTCAACCACAGAATTGCCTTCGATACTGGTTGGCTTGAGACCGGAAGAG-
GACAGCGGAACTGCGAGTGTAGAGGTGAAATTCGTAGATATTCGCAAGAACACCAGTGGCGAAGGCGGCTGTCTGGTCCGGTTCTGACGCTGAGG-
CGCGAAAGCGTGGGGAGCAAACAGGATTAGATACCCTGGTAGTCCACGCCGTAAACGATGAATGCCAGCCGTTGGCCTGCTTGCAGGTCAGTGGCG-
CCGCTAACGCATTAAGCATTCCGCCTGGGGAGTACGGTCGCAAGATTAAAACTCAAAGGAATTGACGGGGGCCCGCACAAGCGGTGGAGCATGTGG-
TTTAATTCGAAGCAACGCGCAGAACCTTACCATCCCTTGACATGGCATGTTACCTCGAGAGATCGGGGATCCTCTTCGGAGGCGTGCACACAGGT-
GCTGCATGGCTGTCGTCAGCTCGTGTCGTGAGATGTTGGGTTAAGTCCCGCAACGAGCGCAACCCACGTCCTTAGTTGCCATCATTTAGTTGGG-
CACTCTAGGGAGACTGCCGGTGATAAGCCGCGAGGAAGGTGTGGATGACGTCAAGTCCTCATGGCCCTTACGGGATGGGCTACACACGTGCTACAA-
TGGCGGTGACAGTGGGACGCGAAGCCGCGAGGTGGAGCAAATCCCCAAAAACCGTCTCAGTTCGGATTGCACTCTGCAACTCGGGTGCATGAAGG-
CGGAATCGCTAGTAATCGTGGATCAGCACGCCACGGTGAATACGTTCCCGGGCCTTGTACACACCGCCCGTCCCCCCATGGGAGTTGGTCTTACCC-
GACGGCGCTGCGCCAACCGCAAGGAGGGCAGGCGACCACGTAGTTACGA - 3'
SAB-1 (Methylobacterium mesophilicum; SEQ.ID.NO 2)

5' TTGGACCTCCGTGGTCGCCTGCCTCCTTGCGGTTGGCGCAGCGCCGTCGGGTAAGACCAACTCCCATGGGGGGACGGCGGTGTGTACAAG-
GCCCGGGAACGTATTCACCGTGGCATGCTGATCCACGATTACTAGCGATTCCGCCTTCATGCACCCGAGTTGCAGAGTGCAATCCGAACTGAGACG-
GCTTTTGGGGATTTGCTCCAGGTCGCCCCTTCGCGTCCCACTGTCACCGCCATTGTAGCACGTGTGTAGCCCATCCCGTAAGGGCCATGAGGACTT-
GACGTCATCCACACCTTCCTCGCGGCTTATCACCGGCAGTCTCCCTAGAGTGCCCAACTGAATGATGGCAACTAAGGACGTGGGTTGCGCTCGTTG-
CGGGACTTAACCCAACATCTCACGACACGAGCTGACGACAGCCATGCAGCACCTGTGTGCGCGCCACCGAAGTGGACTGGGAATCTCTCCCCATAA-
CACGCCATGTCAAAGGATGGTAAGGTTCTGCGCGTTGCTTCGAATTAAACCACATGCTCCACCGCTTGTGCGGGCCCCCGTCAATTCCTTTGAGTT-
TTAATCTTGCGACCGTACTCCCCAGGCGGAATGCTCAAAGCGTTAGCTGCGCTACTGAGGTGCAAGCACCCCAACAGCTGGCATTCATCGTTTACG-
GCGTGGACTACCAGGGTATCTAATCCTGTTTGCTCCCCACGCTTTCGCGCCTCAGCGTCAGTAATGGTCCAGTTGGCCGCCTTCGCCAC-
CGGTGTTCTTGCGAATATCTACGAATTTCACCTCTACACTCGCAGTTCCACCAACCTCTACCATACTCAAGCGTCCCAGTATCGAAGGCCATTCTG-
TGGTTG-AGCCACAGGCTTTCACCCCCGACTTAAAACGCCGCCTACGCGCCCTTTACGCCCAGTGATTCCGAGCAACGCTAGCCCCCTTCGTAT-
TACCGCGGCTGCTGGCACGAAGTTAGCCGGGGCTTATTCCTCCGGTACCGTCATTATCGTCCCGGATAAAAGAGCTTTACAACCCTAAGGCCTT-
CATCACTCACGCGGCATGGCTGGATCAGGCTTGCGCCCATTGTCCAATATTCCCCACTGCTGCCTCCCGTAGGAGTCTGGGCCGTGTCTCAGTCCC-

AGTGTGGCTGATCATCCTCTCAGACCAGCTACTGATCGTCGCCTTGGTGAGCCGTTACCTCACCAACTAGCTAATCAGACGCGGGCCGATCCTCCG-
GCAGCAAGCCTTTCCCCAAAAGGGCGTATCCGGTATTAGCTCAAGTTTCCCTGAGTTATTCCGAACCAGAGGGCACGTTCCCACGCGTTACTCACC-
CGTCCGCCGCTGACACCGAAGGATGCCCCGCTCGACTTGCATGTGTTGACCCCTGCCCCCCAGCGTTCGCTCTGAGCGAGGATACAATCCTCA -
3'

### Beispiel 2: Molekularer Fingerprint der wirksamen Isolate

Zur näheren Charakterisierung und zur Sicherstellung der Wiedererkennbarkeit der wirksamen Isolate wurden von diesen BOX-Muster angefertigt. Diese molekularen Fingerprints sind in der Regel spezifisch für eine lokale Bakterienpopulation und eignen sich somit als individuelle Marker.

### Methode: BOX-Fingerprint

BOX-PCR Fingerprints wurden mit dem BOXA1R Primer 5'-CTA CGG CAA GGC GAC GCT GAC G-3' (SEQ.ID.NO.5) durchgeführt, wozu bakterielle DNA der beiden Methylobacterium-Stämme als Template genutzt wurden. Ein 12 µl Aliquot des amplifizierten PCR-Produkts wurde mittels Gelelectrophorese in einem 1,5%igen Agarosegel aufgetrennt und in 0,5 x TBE für 4 Stunden inkubiert, mit Ethidiumbromid angefärbt und anschließend fotografiert. In Fig. 1 ist das BOX-Muster der wirksamen Isolate dargestellt.

### Beispiel 3: Wirkung der Isolate unter Feldbedingungen

Die wirksamen Isolate wurden in Flüssigkultur vermehrt und anschließend als Zellsuspension in Form eines Wurzeltauchbades vor der Pflanzung bzw. in Form einer Sprühbehandlung nach der Pflanzung an Erdbeerpflanzen der Sorten Senga Sengana und Elsanta im Freiland appliziert. Die von den so behandelten Pflanzen geernteten Erdbeeren wurden durch SPME-GC-Analysen hinsichtlich ihres Gehaltes an Mesifuran untersucht und miteinander verglichen. Aus Fig. 2 wird ersichtlich, dass insbesondere bei der Sorte Senga Sengana durch die Behandlung der Erdbeerpflanzen mit einer Zellsuspension der wirksamen Isolate eine signifikante Erhöhung der Mesifuranwerte in den reifen Früchten erzielt werden kann.

### Methode: Gaschromatographie-Massenspektrometrie (GC-MSD)

Die Bestimmung von Mesifuran als Bestandteil der flüchtigen Verbindungen von Erdbeeren erfolgte mittels Gaschromatographie-Massenspektrometrie (GC-MSD) nach Extraktion der Verbindung aus dem Gasraum über der Probe mittels Solid-Phase-Microextraction (Head SPME).

Headspace SPME ist eine in der Aromastoffanalytik häufig eingesetzte Technik, bei der die flüchtigen Verbindungen aus dem Gasraum über der Probe abgezogen und an einer beschichteten Quarznadel (Faser) angereichert werden. Die anschließende Thermodesorption der Verbindungen von der Faser erfolgt direkt im Injektor des Gaschromatographen, wobei ein direkter Transfer auf die analytische Säule erfolgt.

Die Extraktions- bzw. Anreicherungsbedingungen für Mesifuran waren wie folgt: 5 g Erdbeerpüree wurden mit 2 g NaCl und einem internen Standard (1,2,3 Trichlorpropan, 50 mg/kg) versetzt. Die Extraktion und Anreicherung erfolgte unter Rühren an einer DVB/Carboxen^{™}/PDMS Faser (2 cm, stable flex) bei 45°C über einen Zeitraum von 30 min. Die Thermodesorption erfolgte unmittelbar darauf im GC-Injektor.

Für die analytische Trennung und Detektion von Mesifuran wurden die an der Faser angereicherten Verbindungen im Injektor bei 270°C thermodesorbiert. Zur Verbesserung der Peakform bei der analytischen Trennung wurde am Säulenkopf eine Cryofokussierung durch Einblasen von Flüssigstickstoff vorgenommen. Die anschließende analytische Trennung erfolgte auf einer HP5-Säule (Säulendimensionen 30 m x 0,25 mm x 1 µm). Die Detektion erfolgte mittels massenselektiven Detektors (Elektronenstoßionisation bei 70eV), wobei die Daten einerseits im scan Modus (m/z 35 bis m/z 300) und andererseits im sim Modus mit den für Mesifuran im Massenspektrum charakteristischen Masse-Ladungsverhältnis (m/z 142, 127, 99, 71) aufgenommen wurden. Die Identifizierung der Verbindung erfolgte über das Massenspektrum sowie über die Berechnung des linear temperaturprogrammierten Retentionsindex und Vergleich mit dem Index aus einer Retentionsindexdatenbank ("SKAF").

Der semiquantitative Vergleich der Mesifuran-Mengen in den unterschiedlich behandelten Erdbeerproben erfolgte über den internen Standard. Die Konzentrationen wurden in Äquivalenten zum internen Standard angegeben ("TCP equiv."). Der Response Faktor zwischen Mesifuran und dem internen Standard wurde als 1 angenommen.

Die Ergebnisse sind in Fig. 2 dargestellt (gemessene Mesifuranwerte als Equivalente zu einem Mesifuranstandard, EL = Sorte Elsanta, SS = Sorte Senga Sengana, Elm = Elsanta vom Markt, Elmf = Elsanta vom Markt bei -70°C tiefgefroren, ra = RAB-1, sa = SAB-1, Rh = Rhizostar, SP = Sprühbehandlung, WB = Wurzelbad). Die Sorte Senga Sengana hat höhere Mesifuranwerte als die Sorte Elsanta, die Elsanta vom Markt haben die niedrigsten Mesifuranwerte.

## Patentansprüche

1. Isolat Methylobacterium RAB-1, hinterlegt unter der Hinterlegungsnummer DSM21961 bei der Hinterlegungsstelle DSMZ, und Derivate sowie Mutanten davon.

2. Isolat Methylobacterium SAB-1, hinterlegt unter der Hinterlegungsnummer DSM21962 bei der Hinterlegungsstelle DSMZ, und Derivate sowie Mutanten davon.

3. Präparat enthaltend Isolate, Derivate oder Mutanten gemäß Anspruch 1 und/oder 2, wobei die Isolate, Derivate oder Mutanten in Form von lebenden Zellen, toten Zellen und/oder Fragmenten davon und/oder aus Kulturüberständen derselben bestehen.

4. Präparat nach Anspruch 3, **dadurch gekennzeichnet, dass** es nur entweder RAB-1 oder SAB-1, jedoch keine Mischungen davon, enthält.

5. Präparat nach Anspruch 3 oder 4, **dadurch gekennzeichnet, dass** es Mischungen von RAB-1 oder SAB-1 enthält.

6. Präparat nach einem der Ansprüche 3 bis 5, **dadurch gekennzeichnet, dass** es über Nährstoffe, Nährsubstrate oder Nährflüssigkeiten appliziert wird.

7. Verfahren zur Aromaverbesserung der Erdbeere, **dadurch gekennzeichnet, dass** ein Präparat nach einem der Ansprüche 3 bis 6 auf die Pflanzen oder Pflanzenteile, auf das Pflanzgut, den Samen und/oder in bzw. auf den Boden appliziert wird.

8. Verfahren nach Anspruch 7, **dadurch gekennzeichnet, dass** das Präparat über eine natürliche oder künstliche Matrix appliziert wird.
